(19)

**Europäisches Patentamt**
**European Patent Office**
**Office européen des brevets**

(11) **EP 4 442 723 A1**

(12) **EUROPEAN PATENT APPLICATION**
published in accordance with Art. 153(4) EPC

(43) Date of publication:
**09.10.2024 Bulletin 2024/41**

(21) Application number: **22901762.9**

(22) Date of filing: **30.11.2022**

(51) International Patent Classification (IPC):
*C08G 18/09* (2006.01)     *C07C 311/65* (2006.01)
*C08L 75/02* (2006.01)     *G02B 1/04* (2006.01)

(52) Cooperative Patent Classification (CPC):
**C07C 311/65; C08G 18/09; C08L 75/02; G02B 1/04**

(86) International application number:
**PCT/KR2022/019182**

(87) International publication number:
**WO 2023/101404 (08.06.2023 Gazette 2023/23)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **30.11.2021 KR 20210169322**

(71) Applicant: **Hanwha Solutions Corporation**
**Jung-gu**
**Seoul 04541 (KR)**

(72) Inventors:
• **WOO, Eun Ji**
  **Daejeon 34128 (KR)**
• **PARK, Juyoung**
  **Daejeon 34128 (KR)**
• **SIM, Yujin**
  **Daejeon 34128 (KR)**
• **PARK, Jongseong**
  **Daejeon 34128 (KR)**

(74) Representative: **Berggren Oy**
**P.O. Box 16**
**Eteläinen Rautatiekatu 10A**
**00101 Helsinki (FI)**

(54) **ISOCYANATE COMPOSITION AND PREPARATION METHOD THEREFOR**

(57)     The present disclosure relates to an isocyanate composition that can suppress the occurrence of discoloration and white turbidity and improve transparency when applied to a product, and a preparation method of the same.

**EP 4 442 723 A1**

**Description**

**[TECHNICAL FIELD]**

CROSS-REFERENCE TO RELATED APPLICATION(S)

The present application claims the benefit of priority based on Korean Patent Application No. 10-2021-0169322 filed on November 23, 2021 with the Korean Intellectual Property Office, the disclosure of which is incorporated herein by reference in its entirety.

The present disclosure relates to an isocyanate composition that can suppress the occurrence of discoloration and white turbidity and improve transparency when applied to a product, and a preparation method of the same.

**[BACKGROUND ART]**

Isocyanate is a raw material for polyurethane and is used in various applications such as coatings, cohesive/adhesive agents, paints, foams, and optical materials. In the case of polyurethanes used in fields where excellent appearance properties are required, particularly in optical fields where transparency is required, it is necessary to ensure less discoloration. For this purpose, it is important that no discoloration occurs in the polyurethanization reaction, and also isocyanate as a raw material, especially the difunctional or higher multivalent isocyanate itself, does not cause discoloration.

However, isocyanates have high reactivity, and thus, are oxidized by oxygen or the like in the air during storage process, or is easily deteriorated or discolored by forming self-polymers, which causes occurrence of discoloration or white turbidity even in optical articles such as urethane lenses to which they are applied. Also, during the preparation of polyisocyanate by polymerization of diisocyanate, specifically during the preparation of polyurethane by urethanization reaction of diisocyanate and polyhydric alcohol, the polymer is easily discolored by the catalyst and solvent used in the polymerization reaction.

On the other hand, as a method of suppressing discoloration in isocyanates, polyisocyanates and products produced using the same, various methods have been studied and proposed, such as a method of preparing and storing them by sealing with nitrogen gas and blocking them from air, or a method of storing them by adding additives such as ultraviolet absorbers.

Japanese Unexamined Patent Publication No. (Hei) 2-228317 discloses a method of modifying isocyanate and then treating it with peroxide in the production of light-colored polyisocyanate for polyurethane lacquer, and Japanese Unexamined Patent Publication No. (Hei) 8-291129 discloses a method for preparing isocyanate with reduced discoloration by contacting a discolored isocyanate with an ozone-containing gas. In addition, Japanese Patent Publication No. 2012-506465 discloses a method for preparing isocyanate with reduced discoloration by irradiating the discolored isocyanate with light having a wavelength of 200 to 600 nm.

However, the above methods could not achieve the effect of sufficiently reducing discoloration.

As another method, a method of mixing and storing a compound that is not involved in the polymerization reaction with isocyanate has been proposed, but there was a problem that the added compound caused discoloration during production of subsequent products.

Therefore, there is a need to study a polyisocyanate composition that does not warry about discoloration or deterioration even during the isocyanate synthesis process, storage process, and further product processing process.

**[DETAILED DESCRIPTION OF THE INVENTION]**

**[Technical Problem]**

It is an object of the present disclosure to provide an isocyanate composition that can suppress the occurrence of discoloration and white turbidity and improve transparency when applied to a product, and a preparation method of the same.

**[Technical Solution]**

According to one embodiment of the present disclosure, there is provided an isocyanate composition which comprises an isocyanate-based mixture containing at least one compound selected from the group consisting of monomers, dimers, trimers, and oligomers having 4 to 200 repeating units of isocyanate compounds; phenol; and para-toluenesulfonyl isocyanate, and satisfies the following Equation 1:

[Equation 1]

$$0 < A/50 + B/3 < 1$$

wherein,

A is the Hazen color value (APHA) measured according to ASTMD1003, and
B is the numerical value of the oligomer content relative to the total weight of the isocyanate-based mixture.

According to another embodiment of the present disclosure, there is provided a method for preparing an isocyanate composition, the method comprising the steps of

reacting an amine compound with phosgene in a solvent to obtain an isocyanate-based mixture; and
adding phenol and para-toluenesulfonyl isocyanate to the mixture,
wherein the mixture includes at least one compound selected from the group consisting of monomers, dimers, trimers, and oligomers having 4 to 200 repeating units of isocyanate compounds, and
wherein the isocyanate composition satisfies the following Equation 1:

[Equation 1]

$$0 < A/50 + B/3 < 1$$

wherein,
A is the Hazen color value (APHA) measured according to ASTMD1003, and
B is the numerical value of the oligomer content relative to the total weight of the isocyanate-based mixture.

According to another embodiment of the present disclosure, there is provided a polyisocyanate composition comprising a polyisocyanate formed by a polymerization reaction between the above-mentioned isocyanate composition and a multivalent thiol.
According to yet another embodiment of the present disclosure, there is provided an optical article comprising the above-mentioned polyisocyanate composition.

[Advantageous Effects]

The isocyanate composition according to the present disclosure controls a new parameter that takes into account the content of oligomers in the composition and the Hazen color value of the composition, thereby being able to suppress the occurrence of discoloration and white turbidity in the synthesis process, storage process, and product processing process of the isocyanate composition, and thus improve the transparency of the product.
Therefore, the polyisocyanate composition obtained by polymerizing the isocyanate composition according to the present disclosure can exhibit excellent transparency when applied to an optical article, and particularly exhibit excellent quality when applied to an optical lens.

[DETAILED DESCRIPTION OF THE EMBODIMENTS]

The terms used herein are for the purpose of describing exemplary embodiments only and are not intended to limit the present disclosure. The singular expressions include a plurality of expressions unless expressly stated otherwise in the context. It will be further understood that the terms "comprises", "provides" and/or "has," as used herein specify the presence of stated features, steps, or components, or combinations thereof, but do not exclude the presence or addition of one or more other features, steps, components, or combinations thereof.
The present disclosure can be variously modified and may have various forms, and thus specific embodiments are illustrated and described in detail below. However, the present disclosure is not limited to the specific embodiments and should be construed as including all the changes, equivalents, and substitutions included in the spirit and scope of the present disclosure.
Isocyanate compounds have high reactivity and thus, are easily oxidized by oxygen or the like in the air during storage, or easily deteriorated or discolored by forming self-polymers, which causes a problem that the transparency of optical articles to which they are applied is reduced.

In order to solve these problems, the present inventors have conducted intensive research on the composition of isocyanate compositions, and confirmed that even when lenses are produced from an isocyanate composition that causes yellowing, defects do not occur in some products. The inventors have also found that the transparency of the product is affected by the white turbidity phenomenon in addition to the yellowing of the composition, and when the oligomer content of the isocyanate compound contained in the composition and the Hazen color value of the composition are controlled within a specific range, yellowing and white turbidity can be adjusted within an appropriate range, thus improving the transparency of the final product, and completed the present disclosure.

Specifically, the inventors have found that the isocyanate composition controls a new parameter in which the oligomer content of the isocyanate compound included in the composition and the Hazen color value of the composition are variables, and thus can be applied to an optical article, particularly an optical lens, to realize excellent quality. Particularly, the novel parameter can be realized by appropriately adding phenol and para-toluenesulfonyl isocyanate to an isocyanate-based mixture.

**<Isocyanate Composition>**

The isocyanate composition according to one embodiment of the disclosure comprises an isocyanate-based mixture containing at least one compound selected from the group consisting of monomers, dimers, trimers, and oligomers having 4 to 200 repeating units of isocyanate compounds; phenol; and para-toluenesulfonyl isocyanate.

Here, the isocyanate-based mixture may, in addition to the monomer, which is the isocyanate compound itself, include a dimer in which two isocyanate compounds are bound, a trimer in which three isocyanate compounds are bound, and an oligomer thereof having 4 to 200 repeating units. The oligomer means those in which 4 to 200 isocyanate compounds are bound.

Dimers, trimers, and oligomers of the isocyanate compound may be produced by a side reaction during the synthesis of an isocyanate compound from an amine compound, or may be a by-product in which isocyanate compounds form self-polymers due to their high reactivity. The above by-products may cause not only the transparency and chromaticity of the isocyanate composition itself but also the discoloration and white turbidity of the polyisocyanate composition in which the isocyanate composition is used. Thus, in the case of the isocyanate composition used in the optical field, it is necessary to reduce the content of by-products as described above. However, it is difficult to significantly control the content of byproducts in commercial processes in terms of process efficiency and cost. Therefore, in the present invention, the content of the oligomer in the isocyanate composition is controlled at an appropriate level, thereby being able to improve the transparency and color characteristics of the product while maintaining the efficiency of the process.

In particular, the isocyanate composition includes phenol and para-toluenesulfonyl isocyanate, so that the content of the oligomer in the isocyanate-based mixture and the Hazen color value of the composition can be controlled within an appropriate range, which allows the parameter represented by a novel Equation 1 to be adjusted within a desired range. Here, the para-toluenesulfonyl isocyanate is a component different from the isocyanate-based compound.

The phenol may be contained in an amount of 50 to 1,500 ppm, preferably 100 to 1,000 ppm, based on the total weight of the isocyanate-based mixture. As the phenol is contained in the above range, the content of the oligomer in the mixture and the Hazen color value of the composition can be controlled within an appropriate range, thereby improving the transparency and color characteristics of the product while maintaining the efficiency of the process.

The para-toluenesulfonyl isocyanate may be contained in an amount of 10 to 3,000 ppm, preferably 50 to 2,000 ppm, based on the total weight of the isocyanate-based mixture. As the para-toluenesulfonyl isocyanate is contained in the above range, the content of the oligomer in the mixture and the Hazen color value of the composition can be controlled within an appropriate range, thereby improving the transparency and color characteristics of the product while maintaining the efficiency of the process.

The isocyanate compound is, for example, 1,4-tetramethylene diisocyanate, 1,5-pentamethylene diisocyanate, 1,6-hexamethylene diisocyanate, 1,3-cyclohexylene diisocyanate, 1,4-cyclohexylene diisocyanate, isophorone diisocyanate, norbomene diisocyanate, methylenediphenyl diisocyanate, methylenedicyclohexyl isocyanate, toluene diisocyanate, m-xylylene diisocyanate, p-xylylene diisocyanate and o-xylylene diisocyanate.

In addition, the isocyanate composition according to one embodiment of the present disclosure satisfies the parameters of Equation 1 below. Equation 1 is an index for controlling yellowing and white turbidity phenomena of the composition, and by satisfying Equation 1, it is possible to suppress the occurrence of discoloration and white turbidity in the synthesis process, storage process, and further product processing process, and thus improve the transparency of the product. Specifically, Equation 1 is as follows:

[Equation 1]

$$0 < A/50 + B/3 < 1$$

wherein,

> A is the Hazen color value (APHA) measured according to ASTMD1003, and
> B is the numerical value of the oligomer content relative to the total weight of the isocyanate-based mixture.

Specifically, the content of the oligomer is less than 1.6% by weight based on the total weight of the isocyanate-based mixture. The content of the oligomer is less than 1.6% by weight and thus can reduce the occurrence of yellowing of the composition, and at the same time, by satisfying Equation 1 above, it is possible to effectively control the occurrence of yellowing and white turbidity during production of the product.

On the other hand, if the composition does not satisfy the range of Equation 1 and exhibits a value of 1 or more, the transparency is significantly lowered when applied to an optical lens, the green light appears, and the quality of the product is significantly deteriorated.

Preferably, within the range that satisfies Equation 1, the variable A Hazen color value (APHA) of Equation 1 is more than 0 and less than 50, and the value of the variable B oligomer content in Equation 1 satisfies more than 0 and less than 1.6. By satisfying each of the above ranges, it is possible to significantly improve the transparency and other physical properties when applied to an optical lens.

The variable A Hazen color value (APHA) of Equation 1 can be measured according to ASTM D1003 at normal temperature of $25 \pm 1°C$, and the specific measurement method will be described in more detail in the contents of Experimental Examples.

The numerical value of the amount of oligomer variable B in Equation 1 means only the numerical value excluding the unit from the wt.% of the oligomer. The content of the oligomer can be measured by gel permeation chromatography (GPC) analysis, and the specific measurement method will be described in more detail in the contents of Experimental Examples.

## <Method for preparing isocyanate composition>

A method for preparing an isocyanate composition according to one embodiment of the present disclosure includes a step of reacting an amine compound with phosgene in a solvent to obtain an isocyanate-based mixture; and a step of adding phenol and para-toluenesulfonyl isocyanate to the mixture, thereby satisfying the following Equation 1:

$$[\text{Equation 1}]$$

$$0 < A/50 + B/3 < 1$$

wherein,

> A is the Hazen color value (APHA) measured according to ASTMD1003, and
> B is the numerical value of the oligomer content relative to the total weight of the isocyanate-based mixture.

Particularly, in the step of preparing the isocyanate composition, phenol and para-toluenesulfonyl isocyanate are further added to the reaction mixture, so that the content of oligomers in the mixture and the Hazen color value of the composition can be controlled within an appropriate range, which allows the parameter represented by the novel Equation 1 to be adjusted within a desired range.

The details of Equation 1 can be applied similarly to those described above.

Hereinafter, a method for preparing the isocyanate composition will be described for each step.

First, the method includes a step of reacting an amine compound with phosgene in a solvent to obtain a reaction mixture containing an isocyanate compound.

As the solvent used in the phosgenation reaction, aromatic hydrocarbon solvents such as benzene, toluene, xylene, and ethylbenzene; chlorinated aromatic hydrocarbon solvents such as monochlorobenzene, 1,2-dichlorobenzene, and 1,4-dichlorobenzene; chlorinated hydrocarbon solvents such as dichloromethane, chloroform and carbon tetrachloride, and the like can be used, and two or more of these can be mixed and used.

The amine compound used in the phosgenation reaction may be, for example, an amine compound obtained by hydrogenating a nitro compound, or a chloride thereof. Specifically, the amine compound may be at least one selected from the group consisting of 1,4-tetramethylene diamine, 1,5-pentamethylene diamine, 1,6-hexamethylene diamine, 1,3-cyclohexylene diamine, 1,4-cyclohexylene diamine, isophorone diamine, diamine, methylenediphenyl diamine, methylenedicyclohexyl diamine, toluene diamine, m-xylylene diamine, p-xylylene diamine and o-xylylene diamine, and a salt thereof, and preferably, it may be m-xylylene diamine, p-xylylene diamine, o-xylylene diamine, or a salt thereof.

The amine compound may be contained in an amount of 1 to 20 parts by weight based on 100 parts by weight of the solvent. When the content of the amine compound exceeds 20 parts by weight, a large amount of the amine compound may be precipitated. Preferably, it may be contained in an amount of 1 to 15 parts by weight or 3 to 10 parts by weight.

Specifically, the phosgenation reaction can be performed by a direct phosgenation method in which an amine compound is directly reacted with phosgene (Method 1); a method of reacting an amine compound with anhydrous hydrochloric acid to form an amine-hydrochloride compound, and then reacting the formed salt with phosgene (Method 2); or a method of reacting an amine compound with carbonic acid to form an aliphatic amine-carbonate compound and reacting the formed salt with phosgene (Method 3).

The direct phosgenation method of Method 1 can be performed by reacting an amine compound and phosgene in an organic solvent. At this time, phosgene may be collectively added at the initial stage of the reaction, or after it is partially added at the initial state of the reaction, the remainder may be added dividedly during the reaction.

On the other hand, Method 1 cam be performed by a first step of dissolving a part of phosgene in a solvent and then adding an amine compound thereto; and a second step of adding the remaining phosgene to perform reaction after the completion of the addition of the amine compound. At this time, it is preferable that the first step is performed at a temperature of 15°C to -10 °C so as to prevent toxic phosgene from flowing out and to prevent sudden heat generation during addition of the amine compound, and the phosgenation reaction in the second step may be adjusted to 120 °C to 140°C, so that the reaction can occur at an appropriate reaction rate without worrying about decomposition of the amine compound.

Method 2 can be performed by reacting an amine compound with hydrochloric acid in an organic solvent to form an amine-hydrochloride compound, and then adding phosgene to perform reaction. The formation of the amine-hydrochloride compound can be performed at a temperature of 30 °C or less, preferably at a temperature of about 23 ± 5°C, and the reaction after the addition of phosgene can be adjusted to 120°C to 140°C. When the reaction is performed under such temperature conditions, it is possible to increase the solubility of the amine-hydrochloride compound and prevent thermal decomposition of the isocyanate, thereby preparing a high-purity isocyanate compound in high yield.

Method 3 can be performed by a step of reacting an amine compound with carbonic acid in a solvent to form an amine-carbonate compound, and then adding phosgene to perform reaction. At this time, the formation of the amine-carbonate compound can be performed at a temperature of 30°C or less, preferably at a temperature of about 23 ± 5°C, and the reaction after the addition of phosgene may be adjusted to 80 °C to 180°C. Preferably, the temperature may be in the range of 100 °C or more, or 120 °C or more, and 150 °C or less, or 140 °C or less. When the reaction is performed under such temperature conditions, it is possible to increase the solubility of the amine-carbonate compound and prevent thermal decomposition of the isocyanate, thereby preparing a high-purity isocyanate compound in high yield.

It is preferable to perform the production reaction of the isocyanate compound according to Method 2, because the formation of by-products such as dimers, trimers, and oligomers of the isocyanate compound produced by the phosgenation reaction can be controlled within an appropriate range, and the color of the composition can also be controlled within an appropriate range. This allows the composition to satisfy Equation 1.

After completing the reaction with phosgene in accordance with each Method, a removal step such as nitrogen bubbling for unreacted phosgene and hydrogen chloride gas and a solvent removal process through distillation may be optionally further performed, and these steps may be performed according to conventional methods.

Next, the method includes a step of adding phenol and para-toluenesulfonyl isocyanate to the reaction mixture. When adding additives of the above components, among the components contained in the phosgenation reaction mixture, the content range of the oligomer can be easily controlled, and at the same time, the chromaticity of the composition can be adjusted. Thereby, it is possible to prepare an isocyanate composition that satisfies the parameters of Equation 1 described above.

The phenol may be added in an amount of 50 to 1,500 ppm, preferably 100 to 1,000 ppm, based on the total weight of the isocyanate-based mixture. As the phenol is added in the above range, it is possible to control the content of the oligomer in the mixture and the Hazen color value of the composition within an appropriate range, thereby improving the transparency and color characteristics of the product while maintaining the efficiency of the process.

The para-toluenesulfonyl isocyanate is a compound different from the isocyanate compound synthesized in the step of obtaining the isocyanate-based mixture described above. Here, the para-toluenesulfonyl isocyanate is added in an amount of 10 to 3,000 ppm, preferably 50 to 2,000 ppm, based on the total weight of the isocyanate-based mixture. By adding the para-toluenesulfonyl isocyanate within the above range, it is possible to control the content of the oligomer in the mixture and the Hazen color value of the composition within an appropriate range, thereby improving the transparency and color characteristics of the product while maintaining the efficiency of the process.

The step of adding phenol and para-toluenesulfonyl isocyanate may be performed under nitrogen filling.

<Polyisocyanate Composition>

According to another embodiment of the disclosure, there is provided a polyisocyanate composition comprising a polyisocyanate formed by a polymerization reaction between the above-mentioned isocyanate composition and a multivalent thiol.

The multivalent thiol may be a compound containing two or more thiol groups (-SH) in one molecule, specifically, a compound having two or more, or three or more, and eight or less, or five or less thiol groups in a molecule.

The multivalent thiol compound may be at least one selected from the group consisting of 1,9-dimercapto-3,7-dithianonane, 1,13-dimercapto-3,7,11-trithiatridecane, glycol di(3-mercaptopropionate), 1,4-dithiane-2,5-diyldimethanethiol, 4-mercaptomethyl-1,8-dimercapto-3,6-dithiaoctane, 2-mercaptomethyl-1,5-dimercapto-3-thiapentane, trimethylolpropane tri(3-mercaptopropionate), 4,8-di(mercaptomethyl)-1,11-dimercapto-3,6,9-trithiaundecane, 5,9-di(mercaptoethyl)-1,12-dimercapto-3,7,10-trithiadodecane, pentaerythritol tetra(3-mercaptopropionate), pentaerythritol tetra(mercaptoacetate), 3,6,9,12-tetrathiatetradecane-1,14-dithiol, and 3,6,10,13-tetrathiapentadecane-1,8,15-trithiol.

The polymerization reaction is performed by a thiourethanation reaction between an isocyanate compound in the isocyanate composition and a thiol group in the multivalent thiol.

Specifically, the multivalent thiol may be added in an amount such that the molar ratio of thiol groups in the multivalent thiol to 1 mole of isocyanate groups of the isocyanate compound is 0.8 or more, or 0.9 or more, and 1.1 or less, or 1.0. If the molar ratio of the thiol group to the isocyanate group is less than 0.8 outside the above molar ratio range, the viscosity of the polymer produced is lowered due to excessive isocyanate groups, thereby reducing processability. In addition, when the molar ratio of thiol groups to isocyanate groups exceeds 1.1, the effect of preventing discoloration may be decreased due to excessive thiol groups.

The polymerization reaction may be performed under normal pressure conditions and an inert gas atmosphere such as nitrogen or argon.

It is preferable that the polymerization reaction is performed in a temperature range of - 15°C or more, or 0°C or more, and 20°C or less, or 15°C or less because the reaction rate can be easily controlled without worrying about the occurrence of discoloration, and the reaction efficiency can also be increased.

The polymerization reaction may be performed in the absence of a catalyst, and may be performed in the presence of a catalyst that usually promotes the urethanization reaction, such as a tin-based or amine-based catalyst. When the reaction is performed in the presence of a catalyst, the catalyst may be added together in the step of adding multivalent thiol to the isocyanate-based mixture.

The degree of progress of the polymerization reaction can be predicted by measuring the concentration of isocyanate groups in the polymerization reactant or measuring the refractive index by the n-dibutyl amine method using a differential titration device. In the present disclosure, the reaction may be performed until the concentration of isocyanate groups in the polymerization product reaches the calculated value of isocyanate groups remaining after reacting with the multivalent thiol.

As a result of the polymerization reaction as described above, a polyisocyanate, specifically a polythiourethane, is produced.

The polyisocyanate composition may further include additives such as an internal release agent, an ultraviolet absorber, a polymerization initiator, a heat stabilizer, a color-correcting agent, a chain extender, a crosslinking agent, a light stabilizer, a filler, a photosensitizer, and the like, if necessary. The content can be appropriately determined within a range that does not impair discoloration and discoloration-suppressing properties of the composition.

The polyisocyanate composition described above can be used in a wide range of fields due to its excellent physical properties, and can also be used as an optical material that requires excellent appearance properties, particularly transparency, such as spectacle lenses, camera lenses, plastic lenses, prisms, and the like.

According to yet another embodiment of the disclosure, there is provided an article comprising the polymer composition or made using the polymer composition.

At this time, the article may be an optical article such as a spectacle lens, a camera lens, a plastic lens, or a prism.

Hereinafter, the action and effect of the present disclosure will be described in more detail with reference to specific examples. However, these examples are presented for illustrative purposes only, and the scope of the invention is not defined thereby.

**[Examples and Comparative Examples]**

**Example 1-1**

471 g of 1,2-dichlorobenzene, 32.5 g of m-XDA with a purity of 99.4%, and 0.24 g of 4-hydroxy TEMPO was charged into a flask, and anhydrous hydrochloric acid was injected at a rate of 20 g/hr at room temperature (23±5°C) and stirred. The temperature rose to 50°C while injecting anhydrous hydrochloric acid. After 4 hours of injection, the formed salt was cooled to room temperature, and 43 g of phosgene was charged into a reactor, and heated so that the reactor temperature reached 130°C. From the point of charging of phosgene to the point of completion of the reaction, a dry ice-acetone cooler was used to prevent phosgene from flowing out. After the reactor temperature reached 130°C, the reactor temperature was maintained at 125~135°C for 2 hours so that the reaction solution turned clear. After the solution turned clear, the inside of the reactor was cooled to 80°C. and cooled while purging nitrogen. The solvent was removed from the reaction solution from which phosgene was removed through vacuum distillation, and the product was purified under reduced pressure at a high temperature of 160°C to obtain an isocyanate-based mixture containing m-XDI.

Under nitrogen filling conditions, 100 ppm of phenol and 50 ppm of para-toluenesulfonyl isocyanate were added to the mixture to prepare an isocyanate composition. After preparation, it was stored at 5°C for 180 days.

**Example 1-2**

An isocyanate composition was prepared and stored in the same manner in Example 1-1, except that the content of phenol was changed to 500 ppm.

**Example 1-3**

An isocyanate composition was prepared and stored in the same manner in Example 1-1, except that the content of phenol was changed to 1,000 ppm.

**Example 1-4**

An isocyanate composition was prepared and stored in the same manner in Example 1-1, except that the content of para-toluenesulfonyl isocyanate was changed to 1,000 ppm.

**Example 1-5**

An isocyanate composition was prepared and stored in the same manner in Example 1-1, except that the content of para-toluenesulfonyl isocyanate was changed to 2,000 ppm.

**Example 1-6**

An isocyanate composition was prepared and stored in the same manner in Example 1-2, except that the content of para-toluenesulfonyl isocyanate was changed to 1,000 ppm.

**Example 1-7**

An isocyanate composition was prepared and stored in the same manner in Example 1-2, except that the content of para-toluenesulfonyl isocyanate was changed to 2,000 ppm.

**Example 1-8**

An isocyanate composition was prepared and stored in the same manner in Example 1-3, except that the content of para-toluenesulfonyl isocyanate was changed to 1,000 ppm.

**Example 1-9**

An isocyanate composition was prepared and stored in the same manner in Example 1-3, except that the content of para-toluenesulfonyl isocyanate was changed to 2,000 ppm.

**Comparative Example 1-1**

An isocyanate composition was prepared and stored in the same manner in Example 1-1, except that phenol and para-toluenesulfonyl isocyanate were not added.

**Comparative Example 1-2**

An isocyanate composition was prepared and stored in the same manner in Example 1-1, except that para-toluenesulfonyl isocyanate was not added.

**[Experimental Example 1: Analysis of Isocyanate Composition]**

**(1) Measurement of Hazen color value (APHA)**

For the isocyanate compositions prepared in Examples and Comparative Examples, the Hazen color value (APHA) according to ASTM D1003 was measured, and the results are shown in Table 1 below.
Specifically, the Hazen color value was measured using a xenon lamp light source of Ultrascane equipment (HunterLab) by a method of color difference analysis of the composition under room temperature conditions.

**(2) Gel permeation chromatography (GPC) analysis**

The isocyanate compositions prepared in Examples and Comparative Examples was subjected to gel permeation chromatography (GPC) analysis, and the oligomer content derived thereby is shown in Table 1 below.

<GPC analysis conditions>

Device used: Waters 2988
Columns: Shodex KF-801, KF-802, KF-803 300 mm
Sample concentration: prepared by dissolving 0.1mg of 1wt/vol% sample in 9.9ml of tetrahydrofuran
Carrier: THF
Detection method: UV
Flow rate: 1.0 ml/min
Column temperature: 40 °C
When preparing the calibration curve, polystyrene having a molecular weight of 1000 to 20,000 g/mol was used.

**(3) Derivation of the value of Equation 1**

The value of Equation 1 below was calculated for the isocyanate compositions prepared in Examples and Comparative Examples, and the results are shown in Table 1 below.

$$[\text{Equation 1}]$$

$$0 < A/50 + B/3 < 1$$

wherein,
A is the Hazen color value (APHA) measured according to ASTMD1003, and
B is the numerical value of the oligomer content relative to the total weight of the isocyanate-based mixture.

[Table 1]

| Category | APHA | Oligomer Content | Equation 1 |
|---|---|---|---|
| Example 1-1 Day 0 | 0 | 0.06 | 0.02 |
| Example 1-1 | 23 | 1.06 | 0.81 |
| Example 1-2 | 8 | 1.59 | 0.69 |
| Example 1-3 | 12 | 1.51 | 0.74 |
| Example 14 | 32 | 0.8 | 0.91 |
| Example 1-5 | 45 | 0.27 | 0.99 |

(continued)

| Category | APHA | Oligomer Content | Equation 1 |
|---|---|---|---|
| Example 1-6 | 13 | 0.9 | 0.56 |
| Example 1-7 | 36 | 0.32 | 0.83 |
| Example 1-8 | 9 | 1.3 | 0.61 |
| Example 1-9 | 16 | 0.43 | 0.46 |
| Comparative Example 1-1 | 54 | 0.31 | 1.18 |
| Comparative Example 1-2 | 32 | 2.04 | 1.32 |

**[Example 2 and Comparative Example 2]**

**Example 2-1: Production of Optical Lens**

20.8 g of the isocyanate composition prepared in Example 1-1, 0.04 g of Zelec UN (manufactured by Stepan Company), and 0.04 g of Biosorb 583 (manufactured by Sakai Chemical Industry Co., Ltd) were stirred in a flask at room temperature for about 20 minutes. Next, 0.002 g of dibutyltin chloride was further added and stirred for 10 minutes. 19.2 g of 2,3-bis(2-sulfanyl ethyl sulfanyl)propane-1-thiol was added to the mixture, defoamed at 5 mbar and stirred for 1 hour to prepare a mixed solution.

This mixed solution was filtered through a 1 $\mu$m PTFE filter and then inj ected into a mold composed of a glass mold and tape. The mold was charged into an oven, and the temperature was gradually raised from 10°C to 120°C, and a polymerization reaction was performed for 20 hours. After the polymerization was completed, the mold was taken out of the oven and released to obtain a plastic lens. The obtained plastic lens was annealed at 120°C for 6 hours to produce a final optical lens sample.

**Examples 2-2 to 2-9 and Comparative Examples 2-1 to 2-2**

An optical lens was produced in the same manner in Example 2-1, except that the isocyanate compositions of Examples 1-2 to 1-9 and Comparative Examples 1-1 and 1-2 were used instead of the isocyanate composition of Example 1-1.

**[Experimental Example 2: Optical Lens Evaluation]**

**(1) Color evaluation**

The color of the optical lenses produced in Examples and Comparative Examples was evaluated with the naked eye, and the results are shown in Table 2 below.

**(2) Transparency Evaluation**

For the optical lenses produced in Examples and Comparative Examples, the degree of occurrence of white turbidity was evaluated with the naked eye under various light source conditions according to the following evaluation criteria, and the results are shown in Table 2 below.

<Evaluation Criteria>

C (Clear): Clear under both fluorescent and zirconium lamps
S.H (Slightly lamp haze): Clear under fluorescent light, but some turbidity observed under zirconium lamp
L.H (Lamp Haze): Clear under fluorescent light, but turbidity observed under zirconium lamp
V.H (Visual Haze): Turbidity observed under both fluorescent and zirconium lamps

[Table 2]

| Category | Equation 1 | Color | Transparency |
|---|---|---|---|
| Example 2-1 Day 0 | 0.02 | colorless | C |
| Example 2-1 | 0.81 | colorless | C |
| Example 2-2 | 0.69 | colorless | C |
| Example 2-3 | 0.74 | colorless | C |
| Example 24 | 0.91 | colorless | C |
| Example 2-5 | 0.99 | colorless | C |
| Example 2-6 | 0.56 | colorless | C |
| Example 2-7 | 0.83 | colorless | C |
| Example 2-8 | 0.61 | colorless | C |
| Example 2-9 | 0.46 | colorless | C |
| Comparative Example 2-1 | 1.18 | Mugwort color | C |
| Comparative Example 2-2 | 1.32 | colorless | L.H |

As shown in Table 2, it can be confirmed that an optical lens produced using a composition that satisfies the parameters of Equation 1 defined by the present disclosure is excellent in both chromaticity and transparency compared to Comparative Examples.

**Claims**

1. An isocyanate composition which comprises an isocyanate-based mixture containing at least one compound selected from the group consisting of monomers, dimers, trimers, and oligomers having 4 to 200 repeating units of isocyanate compounds; phenol; and para-toluenesulfonyl isocyanate, and satisfies the following Equation 1:

$$[\text{Equation 1}]$$

$$0 < A/50 + B/3 < 1$$

wherein,

A is the Hazen color value (APHA) measured according to ASTMD1003, and
B is the numerical value of the oligomer content relative to the total weight of the isocyanate-based mixture.

2. The isocyanate composition according to claim 1, wherein:

the A is 0 or more and less than 50, and
the B is more than 0 and less than 1.6.

3. The isocyanate composition according to claim 1, wherein:
the para-toluenesulfonyl isocyanate is different from isocyanate compounds.

4. The isocyanate composition according to claim 1, wherein:

the phenol is contained in an amount of 50 to 1,500 ppm based on the total weight of the isocyanate-based mixture, and
the para-toluenesulfonyl isocyanate is contained in an amount of 10 to 3,000 ppm based on the total weight of the isocyanate-based mixture.

**5.** The isocyanate composition according to claim 1, wherein:
the oligomer is contained in an amount of less than 1.6% by weight based on the total weight of the isocyanate-based mixture.

**6.** The isocyanate composition according to claim 1, wherein:
the isocyanate compound is at least one selected from the group consisting of 1,4-tetramethylene diisocyanate, 1,5-pentamethylene diisocyanate, 1,6-hexamethylene diisocyanate, 1,3-cyclohexylene diisocyanate, 1,4-cyclohexylene diisocyanate, isophorone diisocyanate, norbomene diisocyanate, methylenediphenyl diisocyanate, methylenedicyclohexyl isocyanate, toluene diisocyanate, m-xylylene diisocyanate, p-xylylene diisocyanate and o-xylylene diisocyanate.

**7.** A method for preparing an isocyanate composition, the method comprising the steps of

reacting an amine compound with phosgene in a solvent to obtain an isocyanate-based mixture; and
adding phenol and para-toluenesulfonyl isocyanate to the mixture,
wherein the mixture includes at least one compound selected from the group consisting of monomers, dimers, trimers, and oligomers having 4 to 200 repeating units of isocyanate compounds, and
wherein the isocyanate composition satisfies the following Equation 1:

[Equation 1]

$$0 < A/50 + B/3 < 1$$

wherein,
A is the Hazen color value (APHA) measured according to ASTMD1003, and
B is the numerical value of the oligomer content relative to the total weight of the isocyanate-based mixture.

**8.** The method for preparing an isocyanate composition according to claim 7, wherein:

the A is 0 or more and less than 50, and
the B is more than 0 and less than 1.6.

**9.** The method for preparing an isocyanate composition according to claim 7, wherein:
the para-toluenesulfonyl isocyanate is different from isocyanate compounds.

**10.** The method for preparing an isocyanate composition according to claim 7, wherein:

the phenol is contained in an amount of 50 to 1,500 ppm based on the total weight of the isocyanate-based mixture, and
the para-toluenesulfonyl isocyanate is contained in an amount of 10 to 3,000 ppm based on the total weight of the isocyanate-based mixture.

**11.** The method for preparing an isocyanate composition according to claim 7, wherein:
the oligomer is contained in an amount of less than 1.6% by weight based on the total weight of the isocyanate-based mixture.

**12.** A polyisocyanate composition comprising a polyisocyanate formed by a polymerization reaction between the isocyanate composition as set forth in claim 1 and a multivalent thiol.

**13.** An optical article comprising the polyisocyanate composition as set forth in claim 12.

## INTERNATIONAL SEARCH REPORT

| International application No. |
|---|
| **PCT/KR2022/019182** |

| A. | CLASSIFICATION OF SUBJECT MATTER |
|---|---|

**C08G 18/09**(2006.01)i; **C07C 311/65**(2006.01)i; **C08L 75/02**(2006.01)i; **G02B 1/04**(2006.01)i

According to International Patent Classification (IPC) or to both national classification and IPC

| B. | FIELDS SEARCHED |
|---|---|

Minimum documentation searched (classification system followed by classification symbols)

C08G 18/09(2006.01); B29D 11/00(2006.01); C07C 263/18(2006.01); C08G 18/02(2006.01); C08G 18/08(2006.01); C08G 18/32(2006.01); C08G 18/70(2006.01); C08J 5/00(2006.01); C09J 175/04(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 이소시아네이트(isocyanate), 파라톨루엔설포닐 이소시아네이트(p-toluene sulfonyl isocyanate), 페놀(phenol), 올리고머(oilgomer), 안정성(stability), 싸이올(thiol)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | KR 10-2005-0033045 A (HENKEL KOMMANDITGESELLSCHAFT AUF AKTIEN) 08 April 2005 (2005-04-08)<br>See paragraphs [0007], [0036], [0050] and [0065]; and claim 1. | 1-6 |
| Y | | 7-13 |
| Y | KR 10-2021-0071356 A (SKC CO., LTD. et al.) 16 June 2021 (2021-06-16)<br>See paragraphs [0006] and [0222]-[0240]. | 7-13 |
| A | KR 10-2021-0023483 A (HANWHA SOLUTIONS CORPORATION) 04 March 2021 (2021-03-04)<br>See entire document. | 1-13 |
| A | US 2012-0245258 A1 (SCHMIDT, Hans-Ulrich et al.) 27 September 2012 (2012-09-27)<br>See entire document. | 1-13 |

☑ Further documents are listed in the continuation of Box C.   ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **14 March 2023** | **15 March 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.

**PCT/KR2022/019182**

**C.    DOCUMENTS CONSIDERED TO BE RELEVANT**

| Category* | Citation of document, with indication, where appropriate, of the relevant passages | Relevant to claim No. |
|-----------|-----------------------------------------------------------------------------------|------------------------|
| A | KR 10-1994-0011150 B1 (MITSUI TOATSU CHEMICALS, INC.) 24 November 1994 (1994-11-24)<br>See entire document. | 1-13 |

Form PCT/ISA/210 (second sheet) (July 2022)

INTERNATIONAL SEARCH REPORT
Information on patent family members

International application No.

**PCT/KR2022/019182**

| Patent document cited in search report | | | Publication date (day/month/year) | Patent family member(s) | | | Publication date (day/month/year) |
|---|---|---|---|---|---|---|---|
| KR | 10-2005-0033045 | A | 08 April 2005 | AT | 461949 | T | 15 April 2010 |
| | | | | AU | 2002-347044 | B2 | 21 December 2006 |
| | | | | BR | 0213238 | A | 28 September 2004 |
| | | | | BR | 0213238 | B1 | 27 November 2012 |
| | | | | CA | 2463586 | A1 | 24 April 2003 |
| | | | | CA | 2463586 | C | 21 June 2011 |
| | | | | CN | 100545190 | C | 30 September 2009 |
| | | | | CN | 1568337 | A | 19 January 2005 |
| | | | | DE | 10150722 | A1 | 30 April 2003 |
| | | | | DE | 50214303 | D1 | 06 May 2010 |
| | | | | EP | 1434811 | A1 | 07 July 2004 |
| | | | | EP | 1434811 | B1 | 24 March 2010 |
| | | | | ES | 2341762 | T3 | 28 June 2010 |
| | | | | JP | 2005-505664 | A | 24 February 2005 |
| | | | | KR | 10-0989308 | B1 | 25 October 2010 |
| | | | | PL | 367889 | A1 | 07 March 2005 |
| | | | | US | 2005-0032973 | A1 | 10 February 2005 |
| | | | | US | 9637667 | B2 | 02 May 2017 |
| | | | | WO | 03-033562 | A1 | 24 April 2003 |
| KR | 10-2021-0071356 | A | 16 June 2021 | CN | 112920082 | A | 08 June 2021 |
| | | | | CN | 112920084 | A | 08 June 2021 |
| | | | | CN | 112920374 | A | 08 June 2021 |
| | | | | EP | 3831806 | A1 | 09 June 2021 |
| | | | | JP | 2021-091672 | A | 17 June 2021 |
| | | | | JP | 2021-091893 | A | 17 June 2021 |
| | | | | JP | 7126217 | B2 | 26 August 2022 |
| | | | | KR | 10-2021-0071401 | A | 16 June 2021 |
| | | | | KR | 10-2021-0071798 | A | 16 June 2021 |
| | | | | KR | 10-2021-0071802 | A | 16 June 2021 |
| | | | | KR | 10-2021-0071803 | A | 16 June 2021 |
| | | | | KR | 10-2021-0071811 | A | 16 June 2021 |
| | | | | KR | 10-2021-0071812 | A | 16 June 2021 |
| | | | | KR | 10-2022-0044911 | A | 12 April 2022 |
| | | | | KR | 10-2410626 | B1 | 20 June 2022 |
| | | | | KR | 10-2424204 | B1 | 25 July 2022 |
| | | | | KR | 10-2456416 | B1 | 19 October 2022 |
| | | | | KR | 10-2496434 | B1 | 06 February 2023 |
| | | | | US | 2021-0171447 | A1 | 10 June 2021 |
| | | | | US | 2021-0171448 | A1 | 10 June 2021 |
| | | | | US | 2021-0171452 | A1 | 10 June 2021 |
| KR | 10-2021-0023483 | A | 04 March 2021 | CN | 114269805 | A | 01 April 2022 |
| | | | | EP | 4019564 | A1 | 29 June 2022 |
| | | | | JP | 2022-546351 | A | 04 November 2022 |
| | | | | TW | 202112862 | A | 01 April 2021 |
| | | | | US | 2022-0306835 | A1 | 29 September 2022 |
| | | | | WO | 2021-040316 | A1 | 04 March 2021 |
| US | 2012-0245258 | A1 | 27 September 2012 | AU | 2010-329950 | A1 | 19 July 2012 |
| | | | | AU | 2010-329950 | B2 | 06 November 2014 |
| | | | | BR | 112012013895 | A2 | 03 May 2016 |
| | | | | CA | 2782695 | A1 | 16 June 2011 |

Form PCT/ISA/210 (patent family annex) (July 2022)

<table>
<tr><td colspan="2" align="center">INTERNATIONAL SEARCH REPORT<br>Information on patent family members</td><td colspan="3">International application No.<br><b>PCT/KR2022/019182</b></td></tr>
<tr><td align="center">Patent document<br>cited in search report</td><td align="center">Publication date<br>(day/month/year)</td><td colspan="2" align="center">Patent family member(s)</td><td align="center">Publication date<br>(day/month/year)</td></tr>
<tr><td></td><td></td><td>CN</td><td>102648224   A</td><td>22 August 2012</td></tr>
<tr><td></td><td></td><td>EP</td><td>2510032   A1</td><td>17 October 2012</td></tr>
<tr><td></td><td></td><td>JP</td><td>2013-513007   A</td><td>18 April 2013</td></tr>
<tr><td></td><td></td><td>JP</td><td>5837505   B2</td><td>24 December 2015</td></tr>
<tr><td></td><td></td><td>KR</td><td>10-2012-0107985   A</td><td>04 October 2012</td></tr>
<tr><td></td><td></td><td>MX</td><td>2012006471   A</td><td>28 June 2012</td></tr>
<tr><td></td><td></td><td>MY</td><td>155981   A</td><td>31 December 2015</td></tr>
<tr><td></td><td></td><td>NZ</td><td>601031   A</td><td>28 June 2013</td></tr>
<tr><td></td><td></td><td>WO</td><td>2011-070040   A1</td><td>16 June 2011</td></tr>
<tr><td>KR   10-1994-0011150   B1</td><td>24 November 1994</td><td>EP</td><td>0505150   A1</td><td>23 September 1992</td></tr>
<tr><td></td><td></td><td>EP</td><td>0505150   B1</td><td>14 June 1995</td></tr>
<tr><td></td><td></td><td>JP</td><td>05-078304   A</td><td>30 March 1993</td></tr>
<tr><td></td><td></td><td>JP</td><td>2912490   B2</td><td>28 June 1999</td></tr>
<tr><td></td><td></td><td>US</td><td>5302749   A</td><td>12 April 1994</td></tr>
</table>

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020210169322
- JP 2228317 A
- JP 8291129 A
- JP 2012506465 A